# EUROPÄISCHE PATENTANMELDUNG

(11) **EP 1 029 949 A2**
(43) Veröffentlichungstag der Anmeldung: **23.08.2000**
(21) Anmeldenummer: 00101803.5
(22) Anmeldetag: 28.01.2000
(51) Int. Cl.: C25D 5/10, C25D 5/18, C25D 7/00, A61F 2/06

(54) **Stützstruktur**

(30) Priorität: 16.02.1999 DE 19906417
(71) Anmelder: W.C. Heraeus GmbH & Co. KG, 63450 Hanau (DE)
(72) Erfinder: Herklotz, Günter, Dr., 63486 Bruchköbel (DE); Krüger, Frank, Dr., 61200 Wölfersheim (DE); Frey, Thomas, 63456 Hanau (DE); Giesel, Thomas, 63526 Erlensee (DE)
(74) Vertreter: Kühn, Hans-Christian

(57) **Zusammenfassung**

Es wird unter anderem eine Stützstruktur zur Offenhaltung von Lumina, mit einem Edelstahlsubstrat, bereitgestellt, wobei die Struktur eine die Oberfläche bildende Platinschicht aufweist.

## Beschreibung

Die Erfindung betrifft eine Stützstruktur zur Offenhaltung von Lumina, mit einem Edelstahlsubstrat.

In der Medizintechnik ist es bekannt, Stützstrukturen zur Offenhaltung von Lumina, sogenannte Stents, u. a. aus Edelstahl zu verwenden. Die bei der Aufweitung von solchen Stents auftretenden Torsions- und Biegekräfte stellen hohe Anforderungen an die mechanischen Eigenschaften des Materials. Diese werden von Edelstahl in der Regel erfüllt. Nachteilig hieran ist jedoch die ungenügende Biokompatibilität.

Darüber hinaus wird in EP 0 358 375 auf die Möglichkeit einer Platin-Beschichtung für in der Elektrotechnik im allgemeinen keinen hohen mechanischen Beanspruchungen ausgesetzten Edelstahl-Substrate hingewiesen.

Weiterhin ist es in der Elektrotechnik lediglich bekannt (WO 93/25733), in der Regel keinen hohen mechanischen Beanspruchungen ausgesetzten Bauteile und Substrate mit Platin oder Gold und Platin zu beschichten.

Aus US 5,824,056 wird ein Stent offenbart, der aus einem refraktären Metall und einer mittels Sputtern aufgebrachten biokompatiblen Oberflächenschicht, beispielsweise aus Platin, besteht.

Aus WO 93/07924 A1 ist ein metallisches oder keramisches Implantat bekannt, daß mit einem dünnen infektionsresistenten aufgesputterten Film beschichtet ist.

Bei Schmuckwaren, die im allgemeinen auch keinen hohen mechanischen Beanspruchungen ausgesetzt sind, wird zur Erhöhung der Biokompatibilität, insbesondere zur Vermeidung von allergenen Hautreaktionen eine Vergoldung von Edelstahlschmuckstücken durchgeführt. Jedoch ist auch in diesen Fällen die Biokompatibilität nicht immer ausreichend.

Aus dem vorgenannten ergibt sich das Problem, eine Stützstruktur, insbesondere eine Edelstahl-Stützstruktur, bereitzustellen, die eine ausreichende Biokompatibilität aufweist und der den üblicherweise auftretenden mechanischen Beanspruchungen, insbesondere beim Aufweiten einer Stützstruktur, standhält.

Dieses Problem wird erfindungsgemäß durch eine Stützstruktur nach Anspruch 1 gelöst.

Erfindungswesentlich ist zunächst die überraschende Tatsache, daß die Platinschicht auf dem Edelstahlsubstrat den bei der Applikation auftretenden mechanischen Beanspruchungen standhält. Weiterhin ist es erfindungswesentlich, daß überraschenderweise durch die Anwesenheit einer Goldschicht, die sich zwischen einem Substrat, nämlich einem Edelstahlsubstrat, und der Platinschicht befindet, eine solche Stützstruktur (Stent) den bei der Applikation auftretenden hohen mechanischen Beanspruchungen, insbesondere in Form von Biege- und Torsionskräften, in besonders hohem Maße ohne Ausbildung von relevanten Rissen in der Platinschicht standhält.

Die einzelnen Schichten können mit aus dem Stand der Technik bekannten Verfahren auf die Edelstahloberfläche der jeweiligen Stützstruktur aufgebracht werden, insbesondere mittels aufsputternden PVD-Verfahrens oder mittels galvanischer Abscheidung.

Zur Erhöhung der mechanischen Beanspruchung unter Vermeidung der oben erwähnten Rißbildung ist es vorteilhaft, wenn zwischen der Goldschicht und der Platinschicht eine weitere, zweite Goldschicht angeordnet ist.

Weiterhin sind die folgenden Ausgestaltungen vorteilhaft, da sich diese in der Praxis bewährt haben.

Die erste Goldschicht weist eine Dicke von 0,01 bis 0,2 µm auf. Die zweite Goldschicht weist eine Dicke von 0,5 bis 10,0 µm auf. Die Platinschicht weist eine Dicke von 0,1 bis 1,0 µm auf.

Zur Vermeidung bzw. Verminderung einer bei der galvanischen Abscheidung von Platin auftretenden teilweisen Versprödung durch Aufnahme von Wasserstoff wird in vorteilhafter Weise die Platinschicht mittels Pulsstrom mit Stromumkehr (Reverse-Pulse-Plating) aufgebracht.

Bei diesem Verfahren werden in wechselnder Folge kathodische und anodische Strompulse an dem jeweilig zu platinierenden Werkstück angelegt, um auf diese Art und Weise quasi durch ein "Absprengen" des in situ gebildeten Wasserstoffs von der Metalloberfläche eine möglicherweise auftretende Versprödung des Metalls zu vermeiden.

In der Praxis hat es sich besonders bewährt, wenn die kathodischen Pulse 1,0 bis 100 ms mit einer Stromflußdichte im Bereich von 0,5 bis 10 A/dm² und die anodischen Pulse 0,1 bis 10 ms mit einer Stromdichte im Bereich von 5,0 bis 1.000 A/dm² andauern.

Das nachfolgende Beispiel dient zur Erläuterung der Erfindung.

Ein Edelstahlstent wird mit einem feinen Draht aus Gold oder Edelstahl (Durchmesser ca. 100 µm) kontaktiert und anschließend mit den in der Galvanik üblichen Verfahren entfettet, z.B. kathodisch oder anodisch in einer cyanidischen Lösung. Der so vorbereitete Stent wird in einem salzsauren Goldbad mit einer ersten Goldschicht (Haftgoldschicht) (kathodische Stromdichte ca. 1 - 10 A/dm², Schichtdicke ca. 0,05 µm) versehen.

Anschließend findet eine Beschichtung mit einer zweiten Goldschicht (Feingoldschicht) statt (cyanidisches Goldbad, kathodische Stromdichte ca. 0,1 - 2,0 A/dm², Schichtdicke ca. 0,5 - 8,0 µm).

Wird das Beschichtungsgut im späteren Gebrauch hoher Biegebeanspruchung ausgesetzt - im Fall der Stents ist dies im besonderen Maß gegeben -, so erhöht die zweite Goldschicht die Rißfestigkeit der abschließenden Platinschicht beträchtlich.

Die Platinschicht kann sowohl mit einer Gleichstromabscheidung als auch mit gepulstem Strom aufgebracht werden. Bäder auf der Basis von z.B. Hexachloroplatinat, Hexahydroxoplatinat, Platintetramin, Dinitrodiaminoplatinat sowie schwefelsaure Platinbäder können zur Beschichtung herangezogen werden. Die kathodischen Stromdichten liegen dabei, je nach Bad, zwischen ca. 0,5 und 2,5 A/dm². Findet eine Abscheidung mit gepulstem Strom Anwendung, so hat sich ein Reverse-Pulse-Verfahren bewährt, da auf diese Art und Weise einer zu starken Wasserstoffversprödung der Platinschicht entgegengewirkt werden kann. Eine Pulsfolge, bei der auf einen langen kathodischen Puls (1 - 100 ms) mit niedriger Stromdichte (0,5 - 10 A/dm²) ein kürzerer anodischer Puls (0,1 - 10 ms) mit hoher Stromdichte (5,0 - 1.000 A/dm²) folgt, erbrachte ausgezeichnete Ergebnisse. Eine Strompause (ca. 10 - 1.000 ms) vor dem erneuten kathodischen Puls hat sich bewährt. Die genauen Pulsparameter müssen jedoch an das jeweilige Stentdesign angepaßt werden, da die Stromdichteverteilungen aufgrund von Abschirmungseffekten variieren können.

## Patentansprüche

1. Stützstruktur zur Offenhaltung von Lumina, mit einem Edelstahlsubstrat, dadurch gekennzeichnet, daß die Struktur eine die Oberfläche bildende Platinschicht aufweist und zwischen dem Substrat und der Platinschicht mindestens eine Goldschicht angeordnet ist.

2. Stützstruktur nach Anspruch 1, dadurch gekennzeichnet, daß zwischen der Goldschicht und der Platinschicht eine weitere, zweite Goldschicht angeordnet ist.

3. Stützstruktur nach einem der Ansprüche 1 bis 2, dadurch gekennzeichnet, daß die erste Goldschicht eine Dicke von 0,01 bis 0,2 µm aufweist.

4. Stützstruktur nach einem der Ansprüche 1 bis 3, dadurch gekennzeichnet, daß die zweite Goldschicht eine Dicke von 0,5 bis 10,0 µm aufweist.

5. Stützstruktur nach einem der Ansprüche 1 bis 4, dadurch gekennzeichnet, daß die Platinschicht eine Dicke von 0,1 bis 1,0 µm aufweist.

6. Stützstruktur nach einem der Ansprüche 1 bis 5, dadurch gekennzeichnet, daß die Platinschicht mittels Pulsstrom mit Stromumkehr aufgebracht ist.

7. Stützstruktur nach Anspruch 6, dadurch gekennzeichnet, daß die Struktur beim Beschichten mittels Pulsstrom mit Stromumkehr mit 1 bis 100 ms andauernden kathodischen Strompulsen mit einer Stromdichte im Bereich von 0,5 bis 10 A/dm² beaufschlagt wird.

8. Stützstruktur nach Anspruch 6 oder 7, dadurch gekennzeichnet, daß die Struktur beim Beschichten mittels Pulsstrom mit Stromumkehr mit 0,1 ms bis 10 ms andauernden anodischen Strompulsen mit einer Stromdichte im Bereich von 5,0 bis 1.000 A/dm² beaufschlagt wird.
